# EUROPEAN PATENT SPECIFICATION

(11) **EP 1 532 144 B1**
(45) Date of publication and mention of the grant of the patent: **23.01.2008**
(21) Application number: 03787742.0
(22) Date of filing: 13.08.2003
(51) Int. Cl.: C07D 453/02, A61K 31/439, A61P 25/00

(54) **QUINUCLIDINE DERIVATIVES AND THEIR USE**
CHINUCLEDIN - DERIVATE UND DEREN VERWENDUNG
DERIVES QUINUCLIDINIQUES ET LEUR UTILISATION

(30) Priority: 14.08.2002 DK 200201208; 02.10.2002 DK 200201472
(43) Date of publication of application: 25.05.2005
(73) Proprietor: NEUROSEARCH A/S, 2750 Ballerup (DK)
(72) Inventor: PETERS, Dan, c/o NeuroSearch A/S, 2750 Ballerup (DK); OLSEN, Gunnar M., c/o NeuroSearch A/S, 2750 Ballerup (DK); NIELSEN, Elsebet Ostergaard, c/o NeuroSearch A/S, 2750 Ballerup (DK); AHRING, Philip K., c/o NeuroSearch A/S, 2750 Ballerup (DK); JORGENSEN, Tino Dyhring, c/o NeuroSearch A/S, 2750 Ballerup (DK)
(86) International application number: PCT/DK2003/000538
(87) International publication number: WO 2004/016608

(56) References cited:
- WO-A-98/15551
- WO-A-98/27983
- WO-A-99/31097
- US-A- 5 589 477
- US-A- 5 646 289
- US-A- 5 763 457
- US-A- 5 852 037
- US-A- 5 998 404
- DATABASE STN INTERNATIONAL [Online] File ZCAPLUS, ZCAPLUS accession no. 1996:509522, Document no. 125:167796; YAMANOUCHI PHARMA CO LTD: "Preparation of quinuclidine derivatives as squalene synthase inhibitors" XP002261104 & JP 08 134067 A 28 May 1996 (1996-05-28)

## Description

### TECHNICAL FIELD

This invention relates to novel quinuclidine derivatives and their use as pharmaceuticals. Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

### BACKGROUND ART

The endogenous cholinergic neurotransmitter, acetylcholine, exerts its biological effect via two types of cholinergic receptors, the muscarinic Acetyl Choline Receptors (mAChR) and the nicotinic Acetyl Choline Receptors (nAChR).

It is well established that muscarinic acetylcholine receptors are of importance in relation to memory and cognition, and much research aimed at the development of agents for the treatment of memory related disorders have focused on the synthesis of muscarinic acetylcholine receptor modulators.

Indeed several CNS disorders can be attributed to a cholinergic deficiency, a dopaminergic deficiency, an adrenergic deficiency or a serotonergic deficiency.

*Brown et al.* [Brown et al.: Quinuclidine Inhibitors of 2,3-Oxidosqualene Cyclase-Lanosterol Synthase: Optimization from Lipid Profiles; J. Med. Chem. 1999 42 1306-1311] describe the synthesis of 3-substituted quinuclidine derivatives useful as inhibitors of the cholesterol biosynthesis. An effect on the nicotinic and/or the monoamine receptors is not reported.

US 5,589,477 describes O or N-linked 3-quinuclidine pyrimidine derivatives binding at muscarinic receptors, useful for the treatment of e.g. learning and memory disorders. WO 98/15551 describes O-linked 3-quinuclidine hetero-bicyclic (benzimidazolyl) derivatives being muscarinic antagonists, useful for the treatment of e.g. urinary incontinence, COPD, etc. WO 99/31097 describes O-linked 3-quinuclidine heterocyclic and -bicyclic (imidazolyl and benzimidazolyl) derivatives being muscarinic antagonists, useful for the treatment of e.g. urinary incontinence, COPD, etc. US 5,998,404 describes O or S-linked 1,2,5-oxadiazole and pyrazine derivatives having muscarinic properties. US 5,646,289 describes O or S-linked 1,2,5-thiadiazole derivatives having muscarinic properties. US 5,763,457 describes O or S-linked 3-quinuclidine 1,2,5-oxadiazole derivatives having muscarinic properties. US 5,852,037 describes O or S-linked 3-quinuclidine 1,2,5-thiadiazole derivatives having muscarinic properties. WO 98/27983 describes O or S-linked 3-quinuclidine 1,2,5-thiadiazole derivatives which are squalene synthase inhibitors, useful for the treatment of e.g. undsirable cholesterol levels and diseases of the cardiovascular system, e.g. atherosclerosis. JP 08 134067 describes azabicyclic hetero-bicyclic (dibenzofuran and dibenzothiophen) derivatives being squalene synthase inhibitors, useful as antihyperlipidemics for treating arteriosclerosis.

### SUMMARY OF THE INVENTION

The present invention is devoted to the provision of new quinuclidine derivatives that are modulators of the nicotinic and/or of the monoamine receptors, and which modulators are useful for the treatment of diseases or disorders related to the cholinergic receptors, and in particular the nicotinic acetylcholine receptor, the monoamine receptors, in particular the serotonin receptor (5-HTR), the dopamine receptor (DAR) and the norepinephrine receptor (NER), and of the biogenic amine transporters for serotonin (5-HT), dopamine (DA) and norepinephrine (NE).

Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

The compounds of the invention may also be useful as diagnostic tools or monitoring agents in various diagnostic methods, and in particular for *in vivo* receptor imaging (neuroimaging), and they may be used in labelled or unlabelled form.

Accordingly, in its first aspect the invention provides quinuclidine derivatives represented by Formula I 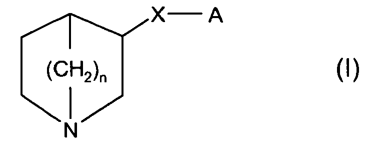 an enantiomer thereof, or a mixture of its enantiomers, or a pharmaceutically-acceptable addition salt thereof, or an onium salt thereof, wherein,
n is 2;
X represents a linker selected from -O-, -O-CH₂- and -O-CH₂-CH₂-; and
A represents a monocyclic heterocyclic group selected from pyridazin-3-yl, thiazol-2-yl, 1,3,4-thiadiazol-2-yl and 1,2,4-thiadiazol-5-yl, which monocyclic heterocyclic group is optionally substituted one or more times with substituents selected from the group consisting of halo, phenyl, 2-thienyl, 3-thienyl, 2-furanyl, 3-furanyl, and 3-pyridinyl, and which phenyl may optionally be substituted one or two times-with halo.

In another aspect the invention provides pharmaceutical compositions comprising a therapeutically effective amount of the quinuclidine derivative of the invention.

In a third aspect the invention relates to the use of the quinuclidine derivative of the invention, or a pharmaceutically-acceptable addition salt thereof, for the manufacture of a pharmaceutical composition/medicament for the treatment, prevention or alleviation of a disease or a disorder or a condition of a mammal, including a human, which disease, disorder or condition is responsive to the action of a nicotinic acetylcholine receptor modulator.

Other objects of the invention will be apparent to the person skilled in the art from the following detailed description and examples.

### DETAILED DISCLOSURE OF THE INVENTION

### Quinuclidine Derivatives

In its first aspect, the present invention provides novel quinuclidine derivatives represented by Formula I 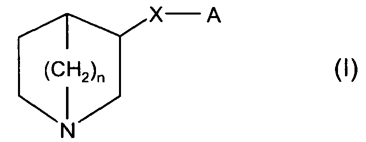 an enantiomer thereof, or a mixture of its enantiomers, or a pharmaceutically-acceptable addition salt thereof, or an onium salt thereof, wherein,
n is 2;
X represents a linker selected from -O-, -O-CH₂- and -O-CH₂-CH₂-; and
A represents a monocyclic heterocyclic group selected from pyridazin-3-yl, thiazol-2-yl, 1,3,4-thiadiazol-2-yl and 1,2,4-thiadiazol-5-yl, which monocyclic heterocyclic group is optionally substituted one or more times with substituents selected from the group consisting of halo, phenyl, 2-thienyl, 3-thienyl, 2-furanyl, 3-furanyl, and 3-pyridinyl, and which phenyl may optionally be substituted one or two times-with halo.

In a most preferred embodiment the quinuclidine derivative of the invention of Formula I is
(±)-3-(5-Bromo-thiazol-2-yloxy)-1-aza-bicyclo[2.2.2]octane;
(±)-3-(5-Phenyl-thiazol-2-yloxy)-1-aza-bicyclo[2.2.2]octane;
(±)-3-[5-(2,4-Difluoro-phenyl)-thiazol-2-yloxy]-1-aza-bicyclo[2.2.2]octane;
(±)-3-[5-(3-Thienyl)-thiazol-2-yloxy]-1-aza-bicyclo[2.2.2]octane;
(±)-3-[5-(2-Thienyl)-thiazol-2-yloxy]-1-aza-bicyclo[2.2.2]octane;
(±)-3-[5-(3-Furanyl)-thiazol-2-yloxy]-1-aza-bicyclo[2.2.2]octane;
(±)-3-[5-(3-Pyridyl)-thiazol-2-yloxy]-1-aza-bicyclo[2.2.2]octane;
(±)-3-(6-Chloro-pyridazin-3-yloxy)-1-aza-bicyclo[2.2.2]octane;
(±)-3-(6-Bromo-pyridazin-3-yloxy)-1-aza-bicyclo[2.2.2]octane;
(±)-3-(6-Phenyl-pyridazin-3-yloxy)-1-aza-bicyclo[2.2.2]octane;
(±)-3-[6-(3-Thienyl)-pyridazin-3-yloxy]-1-aza-bicyclo[2.2.2]octane;
(±)-3-[6-(2-Thienyl)-pyridazin-3-yloxy]-1-aza-bicyclo[2.2.2]octane;
(±)-3-[6-(2-Furanyl)-pyridazin-3-yloxy]-1-aza-bicyclo[2.2.2]octane;
(±)-3-[6-(3-Furanyl)-pyridazin-3-yloxy]-1-aza-bicyclo[2.2.2]octane;
(±)-3-[6-(3-Pyridyl)-pyridazin-3-yloxy]-1-aza-bicyclo[2.2.2]octane;
(±)-3-(5-Phenyl-1,3,4-thiadiazol-2-yloxy)-1-aza-bicyclo[2.2.2]octane;
(±)-3-(5-Phenyl-1,2,4-thiadiazol-3-yloxy)-1-aza-bicyclo[2.2.2]octane; or
(±)-3-[5-(2-Thienyl)-1,3,4-thiadiazol-2-yloxy]-1-aza-bicyclo[2.2.2]octane;
or an enantiomer thereof, or a pharmaceutically-acceptable addition salt thereof, or an onium salt thereof.

Any combination of two or more of the embodiments described herein is considered within the scope of the present invention.

### Definition of Substituents

In the context of this invention halo represents fluoro, chloro, bromo or iodo. Thus a trihalomethyl group represents e.g. a trifluoromethyl group, a trichloromethyl group, and similar trihalo-substituted methyl groups.

### Pharmaceutically Acceptable Salts

The quinuclidine derivative of the invention may be provided in any form suitable for the intended administration. Suitable forms include pharmaceutically (i.e. physiologically) acceptable salts, and pre- or prodrug forms of the quinuclidine derivative of the invention.

Examples of pharmaceutically acceptable addition salts include, without limitation, the non-toxic inorganic and organic acid addition salts such as the hydrochloride derived from hydrochloric acid, the hydrobromide derived from hydrobromic acid, the nitrate derived from nitric acid, the perchlorate derived from perchloric acid, the phosphate derived from phosphoric acid, the sulphate derived from sulphuric acid, the formate derived from formic acid, the acetate derived from acetic acid, the aconate derived from aconitic acid, the ascorbate derived from ascorbic acid, the benzenesulphonate derived from benzensulphonic acid, the benzoate derived from benzoic acid, the cinnamate derived from cinnamic acid, the citrate derived from citric acid, the embonate derived from embonic acid, the enantate derived from enanthic acid, the fumarate derived from fumaric acid, the glutamate derived from glutamic acid, the glycolate derived from glycolic acid, the lactate derived from lactic acid, the maleate derived from maleic acid, the malonate derived from malonic acid, the mandelate derived from mandelic acid, the methanesulphonate derived from methane sulphonic acid, the naphthalene-2-sulphonate derived from naphtalene-2-sulphonic acid, the phthalate derived from phthalic acid, the salicylate derived from salicylic acid, the sorbate derived from sorbic acid, the stearate derived from stearic acid, the succinate derived from succinic acid, the tartrate derived from tartaric acid, the toluene-p-sulphonate derived from p-toluene sulphonic acid, and the like. Such salts may be formed by procedures well known and described in the art.

Other acids such as oxalic acid, which may not be considered pharmaceutically acceptable, may be useful in the preparation of salts useful as intermediates in obtaining a chemical compound of the invention and its pharmaceutically acceptable acid addition salt.

Examples of pharmaceutically acceptable cationic salts of a chemical compound of the invention include, without limitation, the sodium, the potassium, the calcium, the magnesium, the zinc, the aluminium, the lithium, the choline, the lysine, and the ammonium salt, and the like, of a chemical compound of the invention containing an anionic group. Such cationic salts may be formed by procedures well known and described in the art.

In the context of this invention the "onium salts" of N-containing compounds are also contemplated as pharmaceutically acceptable salts (aza-onium salts). Preferred aza-onium salts include the alkyl-onium salts, in particular the methyl- and the ethyl-onium salts; the cycloalkyl-onium salts, in particular the cyclopropyl-onium salts; and the cycloalkylalkyl-onium salts, in particular the cyclopropyl-methyl-onium salts.

### Steric Isomers

The quinuclidine derivatives of the present invention may exist in (+) and (-) forms as well as in racemic forms (±). The racemates of these isomers and the individual isomers themselves are within the scope of the present invention.

Racemic forms can be resolved into the optical antipodes by known methods and techniques. One way of separating the diastereomeric salts is by use of an optically active acid, and liberating the optically active amine compound by treatment with a base. Another method for resolving racemates into the optical antipodes is based upon chromatography on an optical active matrix. Racemic compounds of the present invention can thus be resolved into their optical antipodes, e.g., by fractional crystallisation of d- or I- (tartrates, mandelates, or camphorsulphonate) salts for example.

The quinuclidine derivatives of the present invention may also be resolved by the formation of diastereomeric amides by reaction of the chemical compounds of the present invention with an optically active activated carboxylic acid such as that derived from (+) or (-) phenylalanine, (+) or (-) phenylglycine, (+) or (-) camphanic acid or by the formation of diastereomeric carbamates by reaction of the chemical compound of the present invention with an optically active chloroformate or the like.

Additional methods for the resolving the optical isomers are known in the art. Such methods include those described by Jaques J, Collet A, & Wilen S in "Enantiomers, Racemates, and Resolutions", John Wiley and Sons, New York (1981).

Optical active compounds can also be prepared from optical active starting materials.

### Methods of Preparation

The quinuclidine derivatives of the invention may be prepared by conventional methods for chemical synthesis, e.g. those described in the working examples. The starting materials for the processes described in the present application are known or may readily be prepared by conventional methods from commercially available chemicals.

Also one compound of the invention can be converted to another compound of the invention using conventional methods.

The end products of the reactions described herein may be isolated by conventional techniques, e.g. by extraction, crystallisation, distillation, chromatography, etc.

### Biological Activity

The present invention relates to novel quinuclidine derivatives, which are found to be cholinergic ligands at the nicotinic acetylcholine receptors (nAChR), and modulators of the monoamine receptors, in particular the biogenic amine transporters such as the serotonin receptor (5-HTR), the dopamine receptor (DAR) and the norepinephrine receptor (NER), and of the biogenic amine transporters for serotonin (5-HT), dopamine (DA) and norepinephrine (NE). Also preferred quinuclidine derivatives of the invention show selective α7 activity, as shown in the working examples. The compounds of the present invention may in particular be agonists, partial agonists, antagonists and allosteric modulators of the receptor.

Due to their pharmacological profile the quinuclidine derivatives of the invention may be useful for the treatment of diseases or conditions as diverse as CNS related diseases, PNS related diseases, diseases related to smooth muscle contraction, endocrine disorders, diseases related to neuro-degeneration, diseases related to inflammation, pain, and withdrawal symptoms caused by the termination of abuse of chemical substances.

In a preferred embodiment the quinuclidine derivatives of the invention are used for the treatment of diseases, disorders, or conditions relating to the central nervous system. Such diseases or disorders includes anxiety, cognitive disorders, learning deficit, memory deficits and dysfunction, Alzheimer's disease, attention deficit, attention deficit hyperactivity disorder (ADHD), Parkinson's disease, Huntington's disease, Amyotrophic Lateral Sclerosis, Gilles de la Tourette's syndrome, psychosis, depression, mania, manic depression, schizophrenia, obsessive compulsive disorders (OCD), panic disorders, eating disorders such as anorexia nervosa, bulimia and obesity, narcolepsy, nociception, AIDS-dementia, senile dementia, periferic neuropathy, autism, dyslexia, tardive dyskinesia, hyperkinesia, epilepsy, bulimia, post-traumatic syndrome, social phobia, sleeping disorders, pseudodementia, Ganser's syndrome, pre-menstrual syndrome, late luteal phase syndrome, chronic fatigue syndrome, mutism, trichotillomania, and jet-lag.

In a preferred embodiment diseases, disorders, or conditions relating to the central nervous system for which the quinuclidine derivatives of the invention are used are cognitive disorders, psychosis, schizophrenia and/or depression.

In another preferred embodiment the quinuclidine derivatives of the invention may be useful for the treatment of diseases, disorders, or conditions associated with smooth muscle contractions, including convulsive disorders, angina pectoris, premature labour, convulsions, diarrhoea, asthma, epilepsy, tardive dyskinesia, hyperkinesia, premature ejaculation, and erectile difficulty.

In yet another preferred embodiment the quinuclidine derivatives of the invention may be useful for the treatment of endocrine disorders, such as thyrotoxicosis, pheochromocytoma, hypertension and arrhythmias.

In still another preferred embodiment the quinuclidine derivatives of the invention may be useful for the treatment of neurodegenerative disorders, including transient anoxia and induced neurodegeneration.

In even another preferred embodiment the quinuclidine derivatives of the invention may be useful for the treatment of inflammatory diseases, disorders, or conditions, including inflammatory skin disorders such as acne and rosacea, Chron's disease, inflammatory bowel disease, ulcerative colitis, and diarrhoea.

In still another preferred embodiment the quinuclidine derivatives of the invention may be useful for the treatment of mild, moderate or even severe pain of acute, chronic or recurrent character, as well as pain caused by migraine, postoperative pain, and phantom limb pain. The pain may in particular be neuropathic pain, chronic headache, central pain, pain related to diabetic neuropathy, to post therapeutic neuralgia, or to peripheral nerve injury.

Finally the quinuclidine derivatives of the invention may be useful for the treatment of withdrawal symptoms caused by termination of use of addictive substances. Such addictive substances include nicotine containing products such as tobacco, opioids such as heroin, cocaine and morphine, benzodiazepines and benzodiazepine-like drugs, and alcohol. Withdrawal from addictive substances is in general a traumatic experience characterised by anxiety and frustration, anger, anxiety, difficulties in concentrating, restlessness, decreased heart rate and increased appetite and weight gain.

In this context "treatment" covers treatment, prevention, prophylactics and alleviation of withdrawal symptoms and abstinence as well as treatment resulting in a voluntary diminished intake of the addictive substance.

In another aspect, the quinuclidine derivatives of the invention are used as diagnostic agents, e.g. for the identification and localisation of nicotinic receptors in various tissues.

### Pharmaceutical Compositions

In another aspect the invention provides novel pharmaceutical compositions comprising a therapeutically effective amount of the quinuclidine derivatives of the invention.

While a chemical compound of the invention for use in therapy may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides pharmaceutical compositions comprising the quinuclidine derivative together with one or more pharmaceutically acceptable carriers therefore, and, optionally, other therapeutic and/or prophylactic ingredients, know and used in the art. The carrier(s) must be "acceptable" in the sense of being compatible with the other ingredients of the formulation and not harmful to the recipient thereof.

The pharmaceutical composition of the invention may be administered by any convenient route, which suits the desired therapy. Preferred routes of administration include oral administration, in particular in tablet, in capsule, in dragé, in powder, or in liquid form, and parenteral administration, in particular cutaneous, subcutaneous, intramuscular, or intravenous injection. The pharmaceutical composition of the invention can be manufactured by a person skilled in the art by use of standard methods and conventional techniques appropriate to the desired formulation. When desired, compositions adapted to give sustained release of the active ingredient may be employed.

Further details on techniques for formulation and administration may be found in the latest edition of Remington's Pharmaceutical Sciences (Maack Publishing Co., Easton, PA).

The actual dosage depends on the nature and severity of the disease being treated, and is within the discretion of the physician, and may be varied by titration of the dosage to the particular circumstances of this invention to produce the desired therapeutic effect. However, it is presently contemplated that pharmaceutical compositions containing of from about 0.1 to about 500 mg of active ingredient per individual dose, preferably of from about 1 to about 100 mg, most preferred of from about 1 to about 10 mg, are suitable for therapeutic treatments.

The active ingredient may be administered in one or several doses per day. A satisfactory result can, in certain instances, be obtained at a dosage as low as 0.1 µg/kg i.v. and 1 µg/kg p.o. The upper limit of the dosage range is presently considered to be about 10 mg/kg i.v. and 100 mg/kg p.o. Preferred ranges are from about 0.1 µg/kg to about 10 mg/kg/day i.v., and from about 1 µg/kg to about 100 mg/kg/day p.o.

### EXAMPLES

The invention is further illustrated with reference to the following examples, which are not intended to be in any way limiting to the scope of the invention as claimed.
General remarks: All reactions involving air sensitive reagents or intermediates were performed under nitrogen and in anhydrous solvents. Magnesium sulfate was used as drying agent in the workup-procedures and solvents were evaporated under reduced pressure.

### Method B

### (±)-2-(1-Aza-bicyclo[2.2.2]oct-3-yloxy)-guinoline fumaric acid salt (Compound B1)

A mixture of (±)-3-quinuclidinol (2.0 g, 15.7 mmol), 2-chloroquinoline (2.6 g, 15.7 mmol) and DMF (30 ml) was stirred at room temperature. Sodium hydride (0.94 g, 23.6 mmol, 60% in oil) was added in small portions. The reaction mixture was stirred for 1.5 hours at 50°C. Aqueous sodium hydroxide (50 ml, 1 M) was added followed by extraction with diethyl ether (3 x 50 ml). The combined ethereal phases were washed with aqueous sodium hydroxide (2 x 50 ml, 1 M). The corresponding salt was obtained by addition of a diethyl ether and methanol mixture (9:1) saturated with fumaric acid. Yield 4.62 g (79%). Mp 160.0-160.5°C.

### (±)-3-(6-Phenylpyridazin-3-yloxy)-1-aza-bicyclo[2.2.2]octane fumaric acid salt (Compound B8)

Was prepared according to procedure B from 3-chloro-6-phenylpyridazine. Mp 168.5-172.0°C.

### (±)-3-(5-Phenyl-1,3,4-thiadiazol-2-yloxy)-1-aza-bicyclo[2.2.2]octane fumaric acid salt (Compound B9)

Was prepared according to procedure B from 2-chloro-5-phenyl-1,3,4-thiadiazole. Mp 168.5-172.0°C.

### (±)-3-(5-Bromo-thiazol-2-yloxy)-1-aza-bicyclo[2.2.2]octane fumaric acid salt (Compound B10)

Was prepared according to procedure B from 2,5-dibromothiazole, using 0°C as reaction temperature. Mp 157.8-162.1°C.

### (±)-3-(5-Phenyl-1,2,4-thiadiazol-3-yloxy)-1-aza-bicyclo[2.2.2]octane fumaric acid salt (Compound B12)

Was prepared according to procedure B 3-chloro-5-phenyl-1,2,4,-thiadiazole. Mp 155.0-159.3°C.

### (±)-3-(6-Bromo-pyridazin-3-yloxy)-1-aza-bicyclo[2.2.2]octane fumaric acid salt (Compound B13)

Was prepared according to procedure B from 3,6-dibromopyridazine. Mp 152.8°C.

### (±)-3-(6-Chloro-pyridazin-3-yloxy)-1-aza-bicyclo[2.2.2]octane fumaric acid salt (Compound B14)

Was prepared according to procedure B from 3,6-dichloropyridazine. Mp 164-164.5°C.

### (±)-3-[5-(3-Thienyl)-1,3,4-thiadiazol-2-yloxy]-1-aza-bicyclo[2.2.2]octane fumaric acid salt (Compound B17)

Was prepared according to procedure B from 2-chloro-5-(3-thienyl)-1,3,4-thiadiazole. Mp 186-188°C.

### Method D

### (±)-3-(5-Phenyl-thiazol-2-yloxy)-1-aza-bicyclo[2.2.2]octane fumaric acid salt (Compound D1)

A mixture of (±)-3-(5-bromo-thiazol-2-yloxy)-1-aza-bicyclo[2.2.2]octane (1.25 g, 4.32 mmol), phenylboronic acid (0.791 g, 6.48 mmol), Pd(PPh₃)₄ (0.150 g, 0.13 mmol), aqueous potassium carbonate (6.5 ml, 2 M), 1,3-propanediol (0.97 ml, 13.0 mmol) and 1,2-dimethoxyethane (30 ml) was stirred at reflux for 15 hours. Aqueous sodium hydroxide (50 ml, 1 M) was added, the mixture was extracted with ethyl acetate (3 x 50 ml). Chromatography on silica gel with dichloromethane, methanol and conc. ammonia (89:10:1) gave the title compound. Yield 3.7 g (43%). The corresponding salt was obtained by addition of a diethyl ether and methanol mixture (9:1) saturated with fumaric acid. Mp 170.9-172.2°C.

### (±)-3-[5-(2,4-Difluoro-phenyl)-thiazol-2-yloxy]-1-aza-bicyclo[2.2.2]octane fumaric acid salt (Compound D2)

Was prepared according to method D. Mp 84.3-86.3°C.

### (±)-3-[5-(3-Thienyl)-thiazol-2-yloxy]-1-aza-bicyclo[2.2.2]octane fumaric acid salt (Compound D3)

Was prepared according to method D. Mp 68.5-74.3°C.

### (±)-3-[5-(2-Thienyl)-thiazol-2-yloxy]-1-aza-bicyclo[2.2.2]octane fumaric acid salt (Compound D4)

Was prepared according to method D. Mp 152.6-154.9°C.

### (±)-3-[5-(3-Furanyl)-thiazol-2-yloxy]-1-aza-bicyclo[2.2.2]octane fumaric acid salt (Compound D5)

Was prepared according to method D. Mp 127.6-136.2°C.

### (±)-3-[5-(3-Pyridyl)-thiazol-2-yloxy]-1-aza-bicyclo[2.2.2]octane fumaric acid salt (Compound D6)

Was prepared according to method D. Mp 82.7-86.0°C.

### (±)-3-[6-(3-Thienyl)-pyridazin-3-yloxy]-1-aza-bicyclo[2.2.2]octane fumaric acid salt (Compound D7)

Was prepared according to method D from (±)-3-(6-bromo-pyridazin-3-yloxy)-1-aza-bicyclo[2.2.2]octane. Mp 197.9°C.

### (±)-3-[6-(2-Thienyl)-pyridazin-3-yloxy]-1-aza-bicyclo[2.2.2]octane fumaric acid salt (Compound D8)

Was prepared according to method D from (±)-3-(6-bromo-pyridazin-3-yloxy)-1-aza-bicyclo[2.2.2]octane. Mp 180.3-191.1°C.

### (±)-3-[6-(2-Furanyl)-pyridazin-3-yloxy]-1-aza-bicyclo[2.2.2]octane fumaric acid salt (Compound D9)

Was prepared according to method D from (±)-3-(6-bromo-pyridazin-3-yloxy)-1-aza-bicyclo[2.2.2]octane. Mp 175.8-178.2°C.

### (±)-3-[6-(3-Furanyl)-pyridazin-3-yloxy]-1-aza-bicyclo[2.2.2]octane fumaric acid salt (Compound D10)

Was prepared according to method D from (±)-3-(6-bromo-pyridazin-3-yloxy)-1-aza-bicyclo[2.2.2]octane. Mp 224.8-225.4°C.

### (±)-3-[6-(3-Pyridyl)-pyridazin-3-yloxy]-1-aza-bicyclo[2.2.2]octane fumaric acid salt (Compound D11)

Was prepared according to method D from (±)-3-(6-bromo-pyridazin-3-yloxy)-1-aza-bicyclo[2.2.2]octane. Mp 137.2-143.2°C.

### Biological Activity

### In vitro Inhibition of ³H-α-Bungarotoxine Binding in Rat Brain

In this example the affinity of the compounds of the invention for binding to α₇-subtype of nicotinic receptors is determined.

α-Bungarotoxine is a peptide isolated from the venom of the Elapidae snake *Bungarus multicinctus.* It has high affinity for neuronal and neuromuscular nicotinic receptors, where it acts as a potent antagonist.

³H-α-Bungarotoxine labels nicotinic acetylcholine receptors formed by the α₇ subunit isoform found in brain and the α₁ isoform in the neuromuscular junction.

### Tissue preparation

Preparations are performed at 0-4°C. Cerebral cortices from male Wistar rats (150-250 g) are homogenised for 10 seconds in 15 ml of 20 mM Hepes buffer containing 118 mM NaCl, 4.8 mM KCI, 1.2 mM MgSO₄ and 2.5 mM CaCl₂ (pH 7.5) using an Ultra-Turrax homogeniser. The tissue suspension is subjected to centrifugation at 27,000 x g for 10 minutes. The supernatant is discarded and the pellet is washed twice by centrifugation at 27,000 x g for 10 minutes in 20 ml of fresh buffer, and the final pellet is then re-suspended in fresh buffer containing 0.01% BSA (35 ml per g of original tissue) and used for binding assays.

### Assay

Aliquots of 500 µl of homogenate are added to 25 µl of test solution and 25 µl of ³H-α-bungarotoxine (2 nM, final concentration) and mixed and incubated for 2 hours at 37°C. Non-specific binding is determined using (-)-nicotine (1 mM, final concentration). After incubation, the samples are added 5 ml of ice-cold Hepes buffer containing 0.05% PEI and poured directly onto Whatman GF/C glass fibre filters (pre-soaked in 0.1% PEI for at least 6 hours) under suction, and immediately washed with 2 x 5 ml ice-cold buffer.

The amount of radioactivity on the filters is determined by conventional liquid scintillation counting. Specific binding is total binding minus non-specific binding.

The test value is given as an IC₅₀ (the concentration of the test substance which inhibits the specific binding of ³H-α-bungarotoxin by 50%).

The results of these experiments are presented in Table 1 below.

**Table 1**

| Inhibition of ³H-α-Bungarotoxine Binding | |
|---|---|
| **Compound No.** | **IC₅₀ (µM)** |
| B17 | 0.15 |
| D1 | 0.052 |
| D3 | 0.11 |
| D4 | 0.020 |
| D5 | 0.048 |
| D6 | 0.18 |
| D7 | 0.13 |
| D8 | 0.053 |

## Claims

1. A quinuclidine derivative represented by Formula I 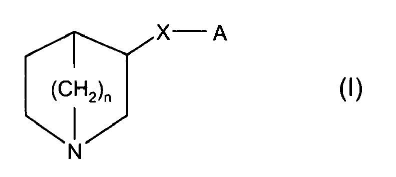
an enantiomer thereof, or a mixture of its enantiomers, or a pharmaceutically-acceptable addition salt thereof, or an onium salt thereof, wherein,
n is 2;
X represents a linker selected from -O-, -O-CH₂- and -O-CH₂-CH₂-; and
A represents a monocyclic heterocyclic group selected from pyridazin-3-yl, thiazol-2-yl, 1,3,4-thiadiazol-2-yl and 1,2,4-thiadiazol-5-yl, which monocyclic heterocyclic group is optionally substituted one or more times with substituents selected from the group consisting of halo, phenyl, 2-thienyl, 3-thienyl, 2-furanyl, 3-furanyl, and 3-pyridinyl, and which phenyl may optionally be substituted one or two times-with halo.

2. The quinuclidine derivative of claim 1, which is
(±)-3-(5-Bromo-thiazol-2-yloxy)-1-aza-bicyclo[2.2.2]octane;
(±)-3-(5-Phenyl-thiazol-2-yloxy)-1-aza-bicyclo[2.2.2]octane;
(±)-3-[5-(2,4-Difluoro-phenyl)-thiazol-2-yloxy]-1-aza-bicyclo[2.2.2]octane;
(±)-3-[5-(3-Thienyl)-thiazol-2-yloxy]-1-aza-bicyclo[2.2.2]octane;
(±)-3-[5-(2-Thienyl)-thiazol-2-yloxy]-1-aza-bicyclo[2.2.2]octane;
(±)-3-[5-(3-Furanyl)-thiazol-2-yloxy]-1-aza-bicyclo[2.2.2]octane;
(±)-3-[5-(3-Pyridyl)-thiazol-2-yloxy]-1-aza-bicyclo[2.2.2]octane;
(±)-3-(6-Chloro-pyridazin-3-yloxy)-1-aza-bicyclo[2.2.2]octane;
(±)-3-(6-Bromo-pyridazin-3-yloxy)-1-aza-bicyclo[2.2.2]octane;
(±)-3-(6-Phenyl-pyridazin-3-yloxy)-1-aza-bicyclo[2.2.2]octane;
(±)-3-[6-(3-Thienyl)-pyridazin-3-yloxy]-1-aza-bicyclo[2.2.2]octane;
(±)-3-[6-(2-Thienyl)-pyridazin-3-yloxy]-1-aza-bicyclo[2.2.2]octane;
(±)-3-[6-(2-Furanyl)-pyridazin-3-yloxy]-1-aza-bicyclo[2.2.2]octane;
(±)-3-[6-(3-Furanyl)-pyridazin-3-yloxy]-1-aza-bicyclo[2.2.2]octane;
(±)-3-[6-(3-Pyridyl)-pyridazin-3-yloxy]-1-aza-bicyclo[2.2.2]octane;
(±)-3-(5-Phenyl-1,3,4-thiadiazol-2-yloxy)-1-aza-bicyclo[2.2.2]octane;
(±)-3-(5-Phenyl-1,2,4-thiadiazol-3-yloxy)-1-aza-bicyclo[2.2.2]octane; or
(±)-3-[5-(2-Thienyl)-1,3,4-thiadiazol-2-yloxy]-1-aza-bicyclo[2.2.2]octane;
or an enantiomer thereof, or a pharmaceutically-acceptable addition salt thereof, or an onium salt thereof.

3. A pharmaceutical composition comprising a therapeutically effective amount of a quinuclidine derivative of either one of claims 1-2, or a pharmaceutically-acceptable addition salt thereof.

4. Use of a quinuclidine derivative of either one of claims 1-2, or a pharmaceutically-acceptable addition salt thereof, for the manufacture of a pharmaceutical composition/medicament for the treatment, prevention or alleviation of a disease or a disorder or a condition of a mammal, including a human, which disease, disorder or condition is responsive to modulation of cholinergic receptors and/or monoamine receptors.

5. The use according to claim 4, wherein the disease, disorder or condition relates to the central nervous system.

6. The use according to claim 5, wherein the disease, disorder or condition is anxiety, cognitive disorders, learning deficit, memory deficits and dysfunction, Alzheimer's disease, attention deficit, attention deficit hyperactivity disorder (ADHD), Parkinson's disease, Huntington's disease, Amyotrophic Lateral Sclerosis, Gilles de la Tourette's syndrome, psychosis, depression, mania, manic depression, schizophrenia, obsessive compulsive disorders (OCD), panic disorders, eating disorders such as anorexia nervosa, bulimia and obesity, narcolepsy, nociception, AIDS-dementia, senile dementia, periferic neuropathy, autism, dyslexia, tardive dyskinesia, hyperkinesia, epilepsy, bulimia, post-traumatic syndrome, social phobia, sleeping disorders, pseudodementia, Ganser's syndrome, pre-menstrual syndrome, late luteal phase syndrome, chronic fatigue syndrome, mutism, trichotillomania, and jet-lag.

7. The use according to claim 4, wherein the disease, disorder or condition are associated with smooth muscle contractions, including convulsive disorders, angina pectoris, premature labour, convulsions, diarrhoea, asthma, epilepsy, tardive dyskinesia, hyperkinesia, premature ejaculation, and erectile difficulty.

8. The use according to claim 4, wherein the disease, disorder or condition is related to the endocrine system, such as thyrotoxicosis, pheochromocytoma, hypertension and arrhythmias.

9. The use according to claim 4, wherein the disease, disorder or condition is a neurodegenerative disorders, including transient anoxia and induced neuro-degeneration.

10. The use according to claim 4, wherein the disease, disorder or condition is an inflammatory disorder, including inflammatory skin disorders such as acne and rosacea, Chron's disease, inflammatory bowel disease, ulcerative colitis, and diarrhoea.

11. The use according to claim 4, wherein the disease, disorder or condition is mild, moderate or even severe pain of acute, chronic or recurrent character, pain caused by migraine, postoperative pain, phantom limb pain, neuropathic pain, chronic headache, central pain, pain related to diabetic neuropathy, to post therapeutic neuralgia, or to peripheral nerve injury.

12. The use according to claim 4, wherein the disease, disorder or condition is associated with withdrawal symptoms caused by termination of use of addictive substances, including nicotine containing products such as tobacco, opioids such as heroin, cocaine and morphine, benzodiazepines and benzodiazepine-like drugs, and alcohol.

## Patentansprüche

1. Chinuclidinderivat, das durch Formel I wiedergegeben wird 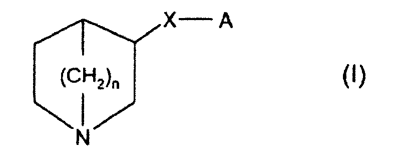
ein Enantiomer davon, oder eine Mischung von seinen Enantiomeren, oder ein pharmazeutisch annehmbares Additionssalz davon, oder ein Oniumsalz davon, wobei
n 2 ist;
X einen Linker, ausgewählt aus -O-, -O-CH₂- und -O-CH₂-CH₂- bedeutet; und
A eine monocyclische heterocyclische Gruppe, ausgewählt aus Pyridazin-3-yl, Thiazol-2-yl, 1,3,4-Thiadiazol-2-yl und 1,2,4-Thiadiazol-5-yl, bedeutet, wobei die monocyclische heterocyclische Gruppe gegebenenfalls ein- oder mehrfach mit Substituenten substituiert ist, die ausgewählt sind aus der Gruppe bestehend aus Halogen, Phenyl, 2-Thienyl, 3-Thienyl, 2-Furanyl, 3-Furanyl und 3-Pyridinyl, und wobei das Phenyl gegebenenfalls ein- oder zweifach mit Halogen substituiert sein kann.

2. Chinuclidinderivat nach Anspruch 1, welches
(±)-3-(5-Brom-thiazol-2-yloxy)-1-aza-bicyclo[2.2.2]octan;
(±)-3-(5-Phenyl-thiazol-2-yloxy)-1-aza-bicyclo[2.2.2]octan;
(±)-3-[5-(2,4-Difluor-phenyl)-thiazol-2-yloxy]-1-aza-bicyclo[2.2.2]octan;
(±)-3-[5-(3-Thienyl)-thiazol-2-yloxy]-1-aza-bicyclo[2.2.2]octan;
(±)-3-[5-(2-Thienyl)-thiazol-2-yloxy]-1-aza-bicyclo[2.2.2]octan;
(±)-3-[5-(3-Furanyl)-thiazol-2-yloxy]-1-aza-bicyclo[2.2.2]octan;
(±)-3-[5-(3-Pyridyl)-thiazol-2-yloxy]-1-aza-bicyclo[2.2.2]octan;
(±)-3-(6-Chlor-pyridazin-3-yloxy)-1-aza-bicyclo[2.2.2]octan;
(±)-3-(6-Brom-pyridazin-3-yloxy)-1 -aza-bicyclo[2.2.2]octan;
(±)-3-(6-Phenyl-pyridazin-3-yloxy)-1-aza-bicyclo[2.2.2]octan;
(±)-3-[6-(3-Thienyl)-pyridazin-3-yloxy]-1-aza-bicyclo[2.2.2]octan;
(±)-3-[6-(2-Thienyl)-pyridazin-3-yloxy]-1-aza-bicyclo[2.2.2]octan;
(±)-3-[6-(2-Furanyl)-pyridazin-3-yloxy]-1 -aza-bicyclo[2.2.2]octan ;
(±)-3-[6-(3-Furanyl)-pyridazin-3-yloxy]-1-aza-bicyclo[2.2.2]octan;
(±)-3-[6-(3-Pyridyl)-pyridazin-3-yloxy]-1-aza-bicyclo[2.2.2]octan;
(±)-3-(5-Phenyl-1,3,4-thiadiazol-2-yloxy)-1-aza-bicyclo[2.2.2]octan;
(±)-3-(5-Phenyl-1,2,4-thiadiazol-3-yloxy)-1-aza-bicyclo[2.2.2]octan; oder
(±)-3-[5-(2-Thienyl)-1,3,4-thiadiazol-2-yloxy]-1-aza-bicyclo[2.2.2]octan ist;
oder ein Enantiomer davon, oder ein pharmazeutisch annehmbares Additionssalz davon, oder ein Oniumsalz davon.

3. Pharmazeutische Zusammensetzung, umfassend eine therapeutisch wirksame Menge eines Chinuclidinderivats nach einem der Ansprüche 1-2 oder eines pharmazeutisch annehmbaren Additionssalzes davon.

4. Verwendung eines Chinuclidinderivats nach einem der Ansprüche 1-2 oder eines pharmazeutisch annehmbaren Additionssalzes davon zur Herstellung einer pharmazeutischen Zusammensetzung/eines Medikaments für die Behandlung, Verhütung oder Linderung einer Krankheit oder einer Störung oder eines Zustands bzw. Leidens eines Säugers, einschließlich eines Menschen, wobei diese Krankheit, diese Störung oder dieser Zustand bzw. dieses Leiden auf die Modulation von cholinergen Rezeptoren und/oder Monoaminrezeptoren anspricht.

5. Verwendung nach Anspruch 4, wobei sich die Krankheit, die Störung oder der Zustand bzw. das Leiden auf das Zentralnervensystem bezieht.

6. Verwendung nach Anspruch 5, wobei es sich bei der Krankheit, der Störung oder dem Zustand bzw. Leiden um Angst, kognitive Störungen, Lerndefizit, Gedächtnisdefizite und -dysfunktion, Alzheimer-Krankheit, Aufmerksamkeitsdefizit, Aufmerksamkeitsdefizit-Hyperaktivitätsstörung (ADHD), Parkinson-Krankheit, Chorea Huntington, amyotrophe Lateralsklerose, Gilles de la Tourette-Syndrom, Psychose, Depression, Manie, manische Depression, Schizophrenie, Zwangsstörungen (OCD), Panikstörungen, Essstörungen wie Anorexia nervosa, Bulimie und Fettleibigkeit, Narkolepsie, Nozizeption, AIDS-Demenz, senile Demenz, periphere Neuropathie, Autismus, Dyslexie, tardive Dyskinesie, Hyperkinesie, Epilepsie, Bulimie, posttraumatisches Syndrom, Sozialphobie, Schlafstörungen, Pseudodemenz, Ganser-Syndrom, prämenstruelles Syndrom, Syndrom der späten Lutealphase, chronisches Ermüdungssyndrom, Mutismus, Trichotillomanie und Jetlag handelt.

7. Verwendung nach Anspruch 4, wobei die Krankheit, die Störung oder der Zustand bzw. das Leiden mit den Kontraktionen glatter Muskeln verbunden ist, einschließlich Krampfstörungen, Angina pectoris, vorzeitiger Wehen bzw. Frühgeburt, Krämpfe, Diarrhoe, Asthma, Epilepsie, tardiver Dyskinesie, Hyperkinesie, vorzeitiger Ejakulation und Erektionsschwierigkeiten.

8. Verwendung nach Anspruch 4, wobei sich die Krankheit, die Störung oder der Zustand bzw. das Leiden auf das endokrine System bezieht, wie etwa Thyreotoxikose, Phäochromozytom, Bluthochdruck und Arrhythmien.

9. Verwendung nach Anspruch 4, wobei die Krankheit, die Störung oder der Zustand bzw. das Leiden eine neurodegenerative Störung ist, einschließlich vorübergehender Anoxie und induzierter Neurodegeneration.

10. Verwendung nach Anspruch 4, wobei es sich bei der Krankheit, der Störung oder dem Zustand bzw. Leiden um eine entzündliche Störung handelt, einschließlich entzündlicher Hautstörungen wie Akne und Rosazea, Morbus Crohn, entzündlicher Darmerkrankung, Colitis ulcerosa und Diarrhoe.

11. Verwendung nach Anspruch 4, wobei es sich bei der Krankheit, der Störung oder dem Zustand bzw. Leiden um milde, mäßige oder auch starke Schmerzen von akutem, chronischem oder wiederkehrendem Charakter, durch Migräne verursachte Schmerzen, postoperative Schmerzen, Phantomschmerzen, neuropathische Schmerzen, chronische Kopfschmerzen, zentrale Schmerzen, mit diabetischer Neuropathie, mit posttherapeutischer Neuralgie oder mit peripherer Nervenschädigung verbundene Schmerzen handelt.

12. Verwendung nach Anspruch 4, wobei die Krankheit, die Störung oder der Zustand bzw. das Leiden mit Entzugssymptomen verbunden ist, die durch die Beendigung der Verwendung von süchtig machenden Substanzen, einschließlich nikotinhaltiger Produkte wie Tabak, Opioide wie Heroin, Kokain und Morphin, Benzodiazepine und Benzodiazepin-artiger Drogen bzw. Arzneimittel und Alkohol, verursacht werden.

## Revendications

1. Dérivé de quinuclidine représenté par la formule I 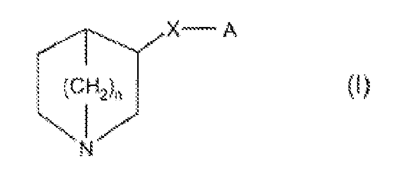
un énantiomère de celui-ci, ou un mélange de ses énantiomères, ou un sel d'addition pharmaceutiquement acceptable de celui-ci, ou un sel d'onium de celui-ci, dans lequel,
n vaut 2 ;
X représente un groupe de liaison choisi parmi -O-, -O-CH₂- et -O-CH₂-CH₂- ; et
A représente un groupe hétérocyclique monocyclique choisi parmi les groupes pyridazin-3-yl, thiazol-2-yl, 1,3,4-thiadiazol-2-yl et 1,2,4-thiadiazol-5-yl, lequel groupe hétérocyclique monocyclique est éventuellement substitué une ou plusieurs fois par des substituants choisis dans le groupe constitué par un halogène, phényle, 2-thiényle, 3-thiényle, 2-furanyle, 3-furanyle, et 3-pyridinyle, et lequel phényle peut être éventuellement substitué une ou deux fois par un halogène.

2. Dérivé de quinuclidine selon la revendication 1, qui est
le (±)-3-(5-bromo-thiazol-2-yloxy)-1-aza-bicyclo[2.2.2]octane ;
le (±)-3-(5-phényl-thiazol-2-yloxy)-1-aza-bicyclo[2.2.2]octane ;
le (±)-3-[5-(2,4-difluoro-phényl)-thiazol-2-yloxy]-1-aza-bicyclo[2.2.2]octane ;
le (±)-3-[5-(3-thiényl)-thiazol-2-yloxy]-1-aza-bicyclo[2.2.2]octane ;
le (±)-3-[5-(2-thiényl)-thiazol-2-yloxy]-1-aza-bicyclo[2.2.2]octane ;
le (±)-3-[5-(3-furanyl)-thiazol-2-yloxy]-1-aza-bicyclo[2.2.2]octane ;
le (±)-3-[5-(3-pyridyl)-thiazol-2-yloxy]-1-aza-bicyclo[2.2.2]octane ;
le (±)-3-(6-chloro-pyridazin-3-yloxy)-1-aza-bicyclo[2.2.2]octane ;
le (±)-3-(6-bromo-pyridazin-3-yloxy)-1-aza-bicyclo[2.2.2]octane ;
le (±)-3-(6-phényl-pyridazin-3-yloxy)-1-aza-bicyclo[2.2.2]octane ;
le (±)-3-[6-(3-thiényl)-pyridazin-3-yloxy]-1-aza-bicyclo[2.2.2]octane ;
le (±)-3-[6-(2-thiényl)-pyridazin-3-yloxy]-1-aza-bicyclo[2.2.2]octane ;
le (±)-3-[6-(2-furanyl)-pyridazin-3-yloxy]-1-aza-bicyclo[2.2.2]octane ;
le (±)-3-[6-(3-furanyl)-pyridazin-3-yloxy]-1-aza-bicyclo[2.2.2]octane ;
le (±)-3-[6-(3-pyridyl)-pyridazin-3-yloxy]-1-aza-bicyclo[2.2.2]octane ;
le (±)-3-(5-phényl-1,3,4-thiadiazol-2-yloxy)-1-aza-bicyclo[2.2.2]octane ;
le (±)-3-(5-phényl-1,2,4-thiadiazol-3-yloxy)-1-aza-bicyclo[2.2.2]octane ; ou
le (±)-3-[5-(2-thiényl)-1,3,4-thiadiazol-2-yloxy]-1-aza-bicyclo[2.2.2]-octane ;
ou un énantiomère de celui-ci, ou un sel d'addition pharmaceutiquement acceptable de celui-ci, ou un sel d'onium de celui-ci.

3. Composition pharmaceutique comprenant une quantité thérapeutiquement efficace d'un dérivé de quinuclidine selon l'une quelconque des revendications 1 ou 2, ou d'un sel d'addition pharmaceutiquement acceptable de celui-ci.

4. Utilisation du dérivé de quinuclidine selon l'une ou l'autre des revendications 1 et 2, ou d'un sel d'addition pharmaceutiquement acceptable de celui-ci, pour la fabrication d'une composition pharmaceutique/d'un médicament destiné(e) au traitement, à la prévention ou au soulagement d'une maladie ou d'un trouble ou d'une condition d'un mammifère, y compris d'un être humain, laquelle maladie, lequel trouble ou laquelle condition est sensible à la modulation des récepteurs cholinergiques et/ou des récepteurs des monoamines.

5. Utilisation selon la revendication 4, dans laquelle la maladie, le trouble ou la condition touche le système nerveux central.

6. Utilisation selon la revendication 5, dans laquelle la maladie, le trouble ou la condition est l'anxiété, les troubles cognitifs, un déficit de l'apprentissage, les troubles et la dysfonction de la mémoire, la maladie d'Alzheimer, un trouble déficitaire de l'attention, un trouble déficitaire de l'attention avec hyperactivité (ADHD), la maladie de Parkinson, la chorée de Huntington, la sclérose latérale amyotrophique, le syndrome de Gilles de la Tourette, la psychose, la dépression, la manie, la psychose maniaco-dépressive, la schizophrénie, les troubles obsessionnels compulsifs (TOC), la panique, les troubles de l'alimentation tels que l'anorexie mentale, la boulimie et l'obésité, la narcolepsie, la nociception, la démence du SIDA, la démence sénile, une neuropathie périphérique, l'autisme, la dyslexie, la dyskinésie tardive, l'hyperkinésie, l'épilepsie, la boulimie, un syndrome post-traumatique, la phobie sociale, les troubles du sommeil, la pseudodémence, le syndrome de Ganser, le syndrome prémenstruel, le syndrome de la phase lutéale tardive, le syndrome de fatigue chronique, le mutisme, la trichotillomanie, et le décalage horaire.

7. Utilisation selon la revendication 4, dans laquelle la maladie, le trouble ou la condition est associé(e) aux contractions des muscles lisses, comprenant les troubles convulsifs, l'angine de poitrine, les contractions prématurées, les convulsions, la diarrhée, l'asthme, l'épilepsie, la dyskinésie tardive, l'hyperkinésie, l'éjaculation précoce, et une difficulté érectile.

8. Utilisation selon la revendication 4, dans laquelle la maladie, le trouble ou la condition touche le système endocrinien, tel que la thyrotoxicose, le phéochromocytome, l'hypertension et les arythmies.

9. Utilisation selon la revendication 4, dans laquelle la maladie, le trouble ou la condition est un trouble neurodégénératif, comprenant l'anoxie temporaire et la neurodégénérescence induite.

10. Utilisation selon la revendication 4, dans laquelle la maladie, le trouble ou la condition est un trouble inflammatoire, comprenant les troubles inflammatoires cutanés tels que l'acné et l'acné rosacée, la maladie de Crohn, l'affection abdominale inflammatoire, la rectocolite hémorragique, et la diarrhée.

11. Utilisation selon la revendication 4, dans laquelle la maladie, le trouble ou la condition est une douleur légère, modérée ou même sévère, de caractère aigu, chronique ou récurrent, causée par la migraine, une douleur postopératoire, une douleur d'un membre fantôme, une douleur neuropathique, une céphalée chronique, une douleur centrale, une douleur liée à une neuropathie diabétique, à une névralgie post-thérapeutique, ou à une lésion d'un nerf périphérique.

12. Utilisation selon la revendication 4, dans laquelle la maladie, le trouble ou la condition est associé(e) aux symptômes de sevrage entraînés par l'interruption de l'utilisation de substances entraînant une dépendance, comprenant les produits contenant de la nicotine tels que le tabac, des opioïdes tels que l'héroïne, la cocaïne et la morphine, les benzodiazépines et les médicaments de type benzodiazépine, et l'alcool.
